# EUROPÄISCHE PATENTANMELDUNG

(11) **EP 0 804 960 A2**
(43) Veröffentlichungstag der Anmeldung: **05.11.1997**
(21) Anmeldenummer: 97106238.5
(22) Anmeldetag: 16.04.1997
(51) Int. Cl.: B01D 61/16, C07C 227/40, C07K 1/36

(54) **Crossflow-Filtrationsverfahren zur Trennung organischer Verbindungen nach Adsorption an anorganischen Feststoffen**

(30) Priorität: 03.05.1996 DE 19617729
(71) Anmelder: Degussa Aktiengesellschaft, 60311 Frankfurt (DE)
(72) Erfinder: Schäfer-Treffenfeldt, Wiltrud, Prof., 63179 Obertshausen (DE); Stockhammer, Stefan, 97662 Bursno (SK); Richet, Gérard, 02100 St. Quentin (FR); Weissland, Günter, 80400 Ham (FR)

(57) **Zusammenfassung**

Verfahren zur Trennung und/oder Abtrennung organischer, in Wasser löslicher Verbindungen, von denen der eine Teil an feinteiligen anorganischen Feststoffen adsorbiert wird, durch Adsorption dieses Anteils und Abtennung des beladenen Festoffs, das dadurch gekennzeichnet ist, daß man
a) die die organischen Verbindungen enthaltenden Lösungen mit einem geeigneten Feststoff in Kontakt bringt,dann eine Crossflow-Filtration durchführt, indem man
b) die entstehenden Suspensionen an einer porösen Fläche/Membran vorbeiströmen läßt, wobei man
c) eine Druckdifferenz zwischen der Überström- und der gegenüberliegenden Seite der Fläche/Membran einstellt,
d) dadurch ein Teil der die Fläche/Membran überströmenden von adsorbierbaren Verbindungen (a) ganz oder teilweise befreiten Lösung die Fläche/Membran quer zur Fließrichtung durchströmt(Filtratfluß)
e) man aus diesem Teil die nicht adsorbierten organischen Verbindungen (b) abtrennt,
f) den die Fläche/Membran nicht durchströmenden Teil der Lösung bzw. Suspension, gegebenenfalls nach Abtrennung eines Teils oder der Gesamtmenge des Feststoffs, wieder in den Kreislauf zur Adsorption der gegebenenfalls noch vorhandenen Restmengen an organischen Verbindungen zurückführt (Punkt a).

## Beschreibung

Die Erfindung betrifft ein Verfahren zur Abtrennung von organischen Verbindungen und/oder Trennung voneinander aus insbesondere wäßrigen Lösungen nach Adsorption an anorganischen Festoffen durch Crossflow-Filtration.

Lösungen bei denen das erfindungsgemäße Verfahren zur Anwendung kommen kann, resultieren z. B. aus der technischen Synthese von Oligopeptiden, bei der immer zum Teil nicht unerhebliche Restkonzentrationen der als Ausgangsstoffe dienenden Aminosäuren zusammen mit dem erwünschten Endprodukt in Lösung verbleiben. Ein entscheidender Schritt bei der Produktion reiner Peptide besteht in der Abtrennung der Ausgangsprodukte. Besonders schwierig ist die Abtrennung der Aminosäuren von den Dipeptiden, wenn zur Herstellung Verfahren benutzt werden, bei denen die für den Schutz der Aminogruppe erforderliche Schutzgruppe nach der Kopplung direkt abgespalten wird (z. B. bei der Peptidkopplung mit N-Carbonsäureanhydriden) und zur Kopplung eine zweite ungeschützte Aminosäure eingesetzt wird. Die in diesen Fällen in Lösung vorliegenden freien Aminosäuren und freien Peptide haben oft sehr ähnliche PKI-Werte und daher ein sehr ähnliches Lösungsverhalten. Eine Reinigung durch Kristallisation ist daher oft nicht oder nur unter großen Verlusten möglich. Für diese Reinigung finden nach dem heutigen Stand der Technik verschiedene chromatographische Verfahren Anwendung.
Bei der Verteilungschromatographie nutzt man die unterschiedlichen Verteilungsgleichgewichte von Aminosäuren und Peptiden zwischen zwei unterschiedlichen Lösungsmittelsystemen (wäßrig und organisch). Liegen diese Verteilungsgleichgewichte nicht ausreichend weit auseinander, wird eine Reinigung nach dieser Methode schwierig bis unmöglich.

Die Affinitätschromatographie, bei der die Unterschiede in den Bindungsstärken zu speziellen Reaktionspartnern ausgenutzt werden, ist nur für sehr kleine Konzentrationen geeignet.
Chromatographieverfahren, die auf hydrophoben Wechselwirkungen zwischen einem Träger und den zu reinigenden Stoffen beruhen, (wie sie auch für Peptide und Proteine in der PCT (SE 93/00582) beschrieben sind) machen sich die Abhängigkeit dieser Wechselwirkungen und damit der Bindung an dem Trägermaterial von der Salzkonzentration im Elutionsmedium zunutze. Bei diesen Verfahren ist oft die Zugabe unpolarer, organischer Substanzen für eine ausreichende Trennleistung notwendig.

Im Vergleich zu den bisher genannten Verfahren hat die Ionenaustausch-Chromatographie die größere Bedeutung in technischen Prozessen erlangt. Die Trennung von Aminosäuren und Peptiden beruht hier auf den Unterschieden der isoelektrischen Punkte der Substanzen. Aminosäuren und Peptide werden bei saurem pH-Wert in ihrer Kationenform durch Ionentausch an einen Kationenaustauscher gebunden.
Die Trennung erfolgt durch Elution mit einem steigenden pH-Gradienten des Elutionsmittels. Bei dem pH-Wert, der ihrem eigenen isoelektrischen Punkt entspricht, werden die einzelnen Aminosäuren und Peptide freigesetzt und eluiert.

Alle genannten chromatographischen Verfahren haben den Nachteil, daß für Substanzen mit ähnlichen und vergleichbaren funktionellen Gruppen mehrere Stufen zur Erzielung einer ausreichenden Reinheit erforderlich sind. Daraus resultiert die Notwendigkeit mehrer Reinigungszyclen, in denen größere Wertstoffverluste nicht vermeidbar sind. Zusätzlich wird durch Elution unter Zugabe von Salzen oder durch pH-Shift die Salzfracht in den Lösungen zum Teil erheblich erhöht. Diese Salze und eventuell auch andere notwendige Zusatzstoffe müssen dann mit erheblichem Aufwand wieder entfernt werden.

Ein weiteres Verfahren, bei dem die Trennung und Reinigung durch Unterschiede im Molekulargewicht und den Abmessungen der zu trennenden Moleküle erfolgt, ist die Gelfiltration. Dabei wird eine poröse Matrix, meist auf organischer Basis, eingesetzt. Größere Moleküle können nicht in die Poren diffundieren und werden schnell eluiert, wohingegen kleinere Moleküle zurückgehalten werden. Von Nachteil ist dabei aber der für einen ausreichenden Durchsatz aufzubringende hohe Druck.

Aus dem Stand der Technik ist auch die sogenannte Crossflow-Mikrofiltration bekannt, mit deren Hilfe man z. B. Bier von Hefe und Trübstoffen reinigt (DE-OS 4401 456).
Bei diesem Verfahren überströmt das ungeklärte oder vorgeklärte Bier in einem Filtermodul eine poröse Membran, so daß sich zwischen der Überström- bzw. Konzentratseite und der gegenüberliegenden Filtratseite der Membran ein transmembraner Druck einstellt.Ein Teil des die Membran überströmenden Bieres durchströmt diese quer und wird als gereinigtes Filtrat auf der Filtratseite gesammelt.
Während der Filtration setzen sich auf der Membranoberfläche die auszufilternden Inhaltsstoffe teilweise als Deckschicht ab. Diese Schicht darf nicht zu kompakt werden, wenn die Durchströmung der Membran während des gesamten Filtrations-prozesses sichergestellt sein soll.
Um dies zu gewährleiten, wird die Filtration in Intervallen unterbrochen und die Deckschicht auf der Membran mit Lauge angelöst.

Dies ist eine Möglichkeit, um der unvermeidlichen Bildung von Deckschichten auf dem Filterkörper zu begegnen.
(Ullmanns's Enzyclopedia of Industrial Chemistry, Vo. B2, Unit Operations 1, 5. Auflage, Seite 10-21)

Aufgabe der Erfindung ist es, ein Verfahren zur Verfügung zu stellen, das eine effektive Trennung von organischen, an anorganischen Feststoffen adsorbierbaren Verbindungen voneinander und insbesondere den aus diesem aufgebauten oder diese enthaltende Verbindungen aus wäßrigen oder wasserhaltigen Lösungen ermöglicht. Bevorzugt werden Lösungen, insbesondere Reaktionsgemische die Di- oder Oligopeptide und die entsprechenden nicht umgesetzten Aminosäuren enthalten, aufgetrennt.

Gegenstand der Erfindung ist ein Verfahren zur Trennung und/oder Abtrennung organischer, in Wasser löslicher Verbindungen, von denen der eine Teil an feinteiligen anorganischen Feststoffen adsorbiert wird, durch Adsorption dieses Anteils und Abtrennung des mit der adsorbierten Verbindung beladenen Feststoffs durch Crossflow-Filtration. Vorzugsweise werden so Lösungen organischer Verbindungen behandelt, die neben den Reaktionsprodukten aus einer Umsetzung die nicht abregierten organischen Ausgangsverbindungen enthalten.
Das Verfahren ist dadurchgekennzeichnet, daß man
a) die die organischen Verbindungen enthaltenden Lösungen mit einem geeigneten Festoff in Kontakt bringt,
b) die entstehenden Suspensionen an einer porösen Fläche/Membran vorbeiströmen läßt,wobei man
c) eine Druckdifferenz zwischen der Überström- und der gegenüberliegenden Seite der Fläche/Membran einstellt,
d) dadurch ein Teil der die Fläche/Membran überströmenden von adsorbierbaren Verbindungen ganz oder teilweise befreiten Lösung die Fläche/Membran quer zur Fließrichtung durchströmt (Filtratfluß),
e) man aus diesem Teil die nicht adsorbierten organischen Verbindungen abtrennt,
f) den die Fläche/Membran nicht durchströmenden Teil der Lösung bzw. Suspension, gegebenenfalls nach Abtrennung eines Teils oder der Gesamtmenge des Feststoffs, wieder in den Kreislauf zur Adsorption der gegebenenfalls noch vorhandenen Restmengen an organischen Verbindungen zurückführt (Punkt a).

Besonders geeignet sind feinteilige Feststoffe, wie z. B. Zeolithpulver, mit einem Partikeldurchmesser von 1 bis 20 µm, insbesondere 2 bis 5 µm.
Diese werden im allgemeinen in Form von wässrigen Suspensionen mit einem Feststoffgehalt von 5 bis 60 Gew.%,bevorzugt 20 bis 50 Gew.-% eingesetzt.

Geringe Gehalte an zu adsorbierenden Verbindungen korrespondieren im allgemeinen mit geringen Feststoffgehalten.
Führt man das Verfahren erfindungsgemäß durch, bildet sich auf dem Filtermedium kein Belag. Somit entstehen auch keine hohen Filtrationswiderstände.
Die Überströmgeschwindigkeiten sind im allgemeinen in einem Bereich von 1 bis 6 m/s, insbesondere 2 bis 4 m/s und der Transmembrandruck in einem Bereich von 0,2 bis 3 bar, insbesondere 0,2 bis 1,6 bar zu variieren.
Dabei zeigt sich, daß der Filtratfluß mit steigender Überströmgeschwindigkeit und zunehmendem Transmembrandruck ansteigt, wobei der letztere Parameter den größeren Einfluß aufweist.

Als Filtermedien sind die aus dem Stand der Technik bekannten für Mikro- und Ultrafiltration geeigneten organischen und anorganischen keramischen Filter.

Insbesondere letztere, wie z. B. in Ullmanns's Enzyclopedia, 5. Auflage (wie oben zitiert) B2, Seite 10-54 abgebildet sind.

Da sich bei den bevorzugt gewählten Filtrationsbedingungen keine Deckschicht bildet, erzielt man erfindungsgemäß durch die konstant hohen Filtratflüsse bereits mit kleinen Filtrationsanlagen kurze Aufarbeitungszeiten.

Aufgabe der Erfindung ist es insbesondere, ein Verfahren zur Verfügung zu stellen, das eine effektive Abtrennung der z. B. nach der Herstellung der Di- oder Oligopeptide in der Lösung verbliebenen, nicht umgesetzten Aminosäuren ermöglicht.

Ein weiterer Gegenstand der Erfindung ist daher ein Verfahren zur Trennung von in Lösung vorliegenden Aminosäuren und/oder Aminosulfonsäuren (a) und diese enthaltende oder aus ihnen hergestellten Produkten (b), insbesondere Dipeptide und/oder Oligopeptide, das dadurch gekennzeichnet ist, daß man die Lösungen bei einem geeigneten pH-Wert mit einem als Adsorbens geeigneten Festoff, insbesondere einem Zeolith, vorzugsweise der Typen TA, FAU, MOR oder MSI gegebenenfalls in mehreren Schritten in Kontakt bringt, und die in der Lösung verbliebenen Produkte von den an dem Feststoff adsorbierten Aminosäuren und/oder Aminosulfonsäuren durch Crossflow-Filtration abtrennt.

Als Adsorbentien sind ebenso geeignet Titansilikalit oder anorganische Verbindungen des Typs M 41 S oder MCM-41, die eine regelmäßige Mesoporenstruktur (20 - 100 Å) aufweisen (US-PS 5,098,684, J.L. Casci, Advanced Zeolite Science and Applications (Stud. Surf.Sci.catal. 95 (1994) 329 ff.)

Die Adsorptionstemperatur kann dabei zwischen den Schmelz- und Siedepunkten des verwendeten Lösungsmittels, vorzugsweise zwischen 15 und 35 °C liegen.
Bei den abzutrennenden Aminosäuren handelt es sich vorzugsweise um organischen Verbindungen, die jeweils mindestens eine Aminogruppe sowie eine Carbonsäure- oder eine Sulfonsäuregruppe enthalten. Bevorzugt sind Aminosäuren und/oder Aminosulfonsäuren, bei denen die Amino- und Säuregruppe über einem C₁- C₄ -Alkylenrest verbunden sind.

Falls die Aminosäuren oder Aminosulfonsäuren ein chirales Zentrum enthalten, ist das Verfahren auf beide Enantiomere anwendbar. Bei den Aminogruppen kann es sich um primäre, sekundäre oder tertiäre Amingruppen handeln.
Die mit dem erfindungemäßen Verfahren abtrennbaren Aminosäuren und Aminosulfonsäuren können noch weitere, funktionelle Gruppen, wie z. B.
Carboxyl, Sulfenyl, Hydroxyl, Amino- Thionyl, Guanidin; Heteroaryl tragen. Diese Gruppen können gegebenenfalls auch mit in der Peptidchemie gebräuchlichen Schutzgruppen, wie z. B. Benzyloxycarboxyl-, t-Butoxycarboxyl, Trifluoracetyl-, Tosyl-für Aminogruppen bzw. Guanidinogruppen oder Alkylester für Carboxylgruppen tragen.
Das Verfahren läßt sich besonders vorteilhaft zur Abtrennung von Aminosäuren und/oder Aminosulfonsäuren von diese enthaltende oder aus ihnen hergestellten Produkten anwenden, wenn die Produkte ebenfalls freie Amino- und Carbonsäure- bzw. Sulfonsäuregruppen enthalten. Bevorzugt wird das Verfahren zur Abtrennung von nichtumgesetzten Aminosäuren und/oder Aminosulfonsäuren von Produkten eingesetzt, die durch die Verknüfung von zwei oder mehr Aminosäuren hergestellt werden. Besonders bevorzugt sind Verbindungen, bei denen die Verknüpfung über Amidbindungen erfolgt.

Bevorzugt wird das erfindungsgemäße Verfahren zur Abtrennung von Di- und/oder Tripeptiden aus überwiegend wäßrigen Lösungen angewendet, in denen die die Peptide bildenden Aminosäuren, bzw. Aminosulfonsäuren sämtlich oder zum Teil enthalten sind.

Beispielhaft angeführt seien die vorzugsweise durchgeführten Trennungen von Dipeptiden und den in ihnen enthaltenen α-L-Aminosäuren:
Ala + Pro von Ala-Pro
Val + Pro von Val-Pro
Gly + Gln von Gly-Gln
Tyr + Arg von Tyr-Arg
Gly + Glu von Gly-Glu

Als ein Beispiel zur Abtrennung von β-L-Aminosäuren ist anzusehen:
β-Ala + His von β-Ala-His

Dasselbe gilt für die Abtrennung von α-L-Aminosäuren aus Tripeptide enthaltenden Lösungen:
Gly + Tyr von Gly-Gly-Tyr.

Als ein Beispiel für die Abtennung einer Diaminocarbonsäure, bei der eine Aminogruppe durch eine Trifluoracetylgruppe geschützt ist, ist zu nennen:
ε-TFA - Lys + Pro von ε-TFA-Lys-Pro
(TFA: Trifluoracetyl)

Die praxisnahe Prüfung des erfindungsgemäßen Verfahrens zeigt die universelle Anwendbarkeit, obwohl Aminosäuren eine Vielzahl von unterschiedlichen chemischen Eigenschaften besitzen können.

Entsprechend weisen die aus proteinogenen Aminosäuren bestehenden Peptide
a) 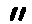 hydrophobe" Seitenketten: *Gly*, *Ala*, *Pro*, *Val* oder/und
b) 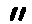 polare" Seitenketten: *Tyr*, *Gln* oder/und
c) 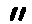 saure" Seitenketten: *Glu* oder/und
d) 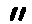 basische" Seitenketten: *Arg*, *Lys*
auf, wie anhand der aufgeführten Aminosäuren beispielhaft belegt wird.

Erfindungsgemäß gelingt auch die Abtrennung von Aminosäuren mit sekundären Amino-Gruppen, wie z. B.
*Sar* + *Ala* von *Sar-Ala (*Sar = Sarkosin).

Dasselbe gilt auch für die Abtrennung von Aminosulfonsäuren,wie z. B.
*Ala* + *Tau von Ala-Tau* (Tau = Taurin)

Da die Adsorption von Aminosäuren im gesamten pH-Bereich zwischen 1 und dem jeweiligen IP möglich ist, wird in Lösungen mit dem entsprechenden pH-Wert keine pH-Korrektur notwendig.

Vorzugsweise führt man die Adsorption bei einem pH <IP (Isoelektrischer Punkt) durch, während die Desorption vorzugsweise bei einem pH >IP vorgenommen wird.
Liegen die isoelektrischen Punkte zweier aus der Lösung zu entfernender Aminosäuren bzw. Aminosulfonsäuren deutlich auseinander, wird der Adsorptionsschritt gegebenenfalls bei unterschiedlichen pH-Werten wiederholt.

Das Verfahren eignet sich auch sehr gut für die Trennung von in einer gemeinsamen Lösung vorliegendem L-Leucin und L-Isoleucin voneinander. Der das adsorbierte L-Leucin enthaltende Zeolith wird über das Cross-Flow-Verfahren von dem in Lösung verbleibenden L-Isoleucin abgetrennt (s. a. EP-A-0645 371).

Als Zeolithe werden erfindungsgemäß bevorzugt solche der Typen FAU, MSI oder Mordenit eingesetzt,die einen Modul von 15 bis 200 besitzen.
Die Zeolithe als solche sind aus dem Stand der Technik bekannt.

Die zu reinigende Lösung wird beispielsweise mit dem Zeolithen durch direkte Zugabe von Zeolithpulver zur Lösung in einem durchmischten Behälter in Kontakt gebracht. Das Abtrennen der gereinigten Wertstofflösung vom Zeolithpulver erfolgt durch anschließende Crossflow-Filtration.

Der mit Aminosäuren beladene Feststoff kann, wenn die abgetrennten Aminosäuren nicht zurückgewonnen werden sollen, durch Erhitzen bei Temperaturen zwischen 400 und 900 °C, z. B. in einem Drehrohrofen, regeneriert werden.
Die adsorbierten Aminosäuren können aber auch in wäßrigen Lösungen bei pH-Werten die in Abhängigkeit von den jeweiligen Aminosäuren eingestellt werden, vollständig desorbiert und durch die Crossflow-Filtration von dem Zeolithpulver getrennt, dadurch wiedergewonnen werden. Der Festostoff kann danach erneut zur Reinigung von Peptiden eingesetzt werden.

Aufgrund der günstigen Lage des Adsorptionsgleichgewichtes gelingt es, die Aminosäuren bzw. Aminosulfonsäuren, bei Zugabe einer entsprechenden Zeolithmenge, in einem Schritt nahezu vollständig aus der Lösung zu entfernen. Selbst wenn in manchen Fällen dabei auch der Wertstoff in geringen Mengen adsorbiert wird, bleibt immer eine deutliche Trennleistung zwischen freien Aminosäuren und Wertstoff zu beobachten.

Im Vergleich zum Stand der Technik zeichnet dies -unter anderem- das erfindungsgemäße Verfahren aus.

Dazu kommt die hohe Effizienz, da bei der Abtrennung geringer Mengen an Nebenprodukten bzw. Ausgangsverbindungen aus Reaktionslösungen, wie sie bevorzugt eingesetzt werden, von den zu reinigenden Wertstoffen im Vergleich zur Ionenaustauscher-Chromatographie keine Kapazität des Adsorptionsmittels durch den Wertstoff (hier Peptide) beansprucht wird.
Die benötigte Adsorbensmenge richtet sich allein nach der Menge der zu entfernenden Substanzen.

Als vorteilhaft erweist sich, daß erfindungsgemäß kein Aufsalzen und nur eine im Vergleich zum Stand der Technik (Trennung über organische Ionenaustauscher) geringe Verdünnung der Wertstofflösung notwendig ist.

Die Konzentration der gewünschten Produkte erstreckt sich in Abhängigkeit von ihrer Löslichkeit und den nach dem jeweils gewählten Verfahren erzielbaren Konzentrationswerten im allgemeinen auf einen Bereich von 1 Gew.-% bis 60 Gew.-%, bezogen auf die sie enthaltende Lösung, insbesondere 4 bis 30 Gew.-%.

Die Konzentration der abzutrennenden, z. B. als Ausgangsverbindungen dienenden Aminosäuren beläuft sich im allgemeinen auf eine nach der Umsetzung aufzufindende Restkonzentration von 0,01 g/l bis auf den Wert, der durch die Löslichkeitsgrenze der jeweils eingesetzten Ausgangsverbindung vorgegeben ist.
Drückt man das Verhältnis der einzelnen abzutrennenden Verbindung, wie z. B. der oder den Aminosäure(n) zu dem zu reinigenden Produkt in g/l aus, findet man einen Bereich von 1 : 1000 bis 1 : 1,5 insbesondere 1 : 300 bis 1 : 1,5, in dem das erfindungsgemäße Verfahren erfolgreich anzuwenden ist.

Es zeigt sich bei dem erfindungsgemäßen Verfahren, daß auch mit 20 Gew.-%igen Feststoffsuspensionen ein Filtratfluß von ca. 500 l/m²h erreicht werden kann. Dieser sehr hohe Fluß entspricht fast dem reinen Wassers. Dies konnte nicht erwartet werden.

Dies ist auch eine Folge des Umstands, daß sich erfindungsgemäß keine Deckschicht auf dem Filtermodul bildet.
Man arbeitet erfindungsgemäß im allgemeinen im Bereich von 20 bis 500 l/m²h Filtratfluß.

Die Charakterisierung der verwendeten Zeolithe entspricht der Einordnung nach W.M. Meier, D.H. Olson 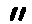 Atlas of Zeolite Structure Types, 2nd Ed. Butterworth-Heinemann, London, 1987.

Das gilt insbesondere für
- Zeolith A: ^ Zeolith TA
- Zeolith DAY: ^ Zeolith FAU
- Mordenit: ^ MOR
- ZSM 5: ^ MSI

Die zur Kennzeichnung des ZSM 5 Typs durch Bindestrich verbundene Zahl in den Beispielen entspricht dem jeweiligen SiO₂/Al₂O₃-Verhältnis.

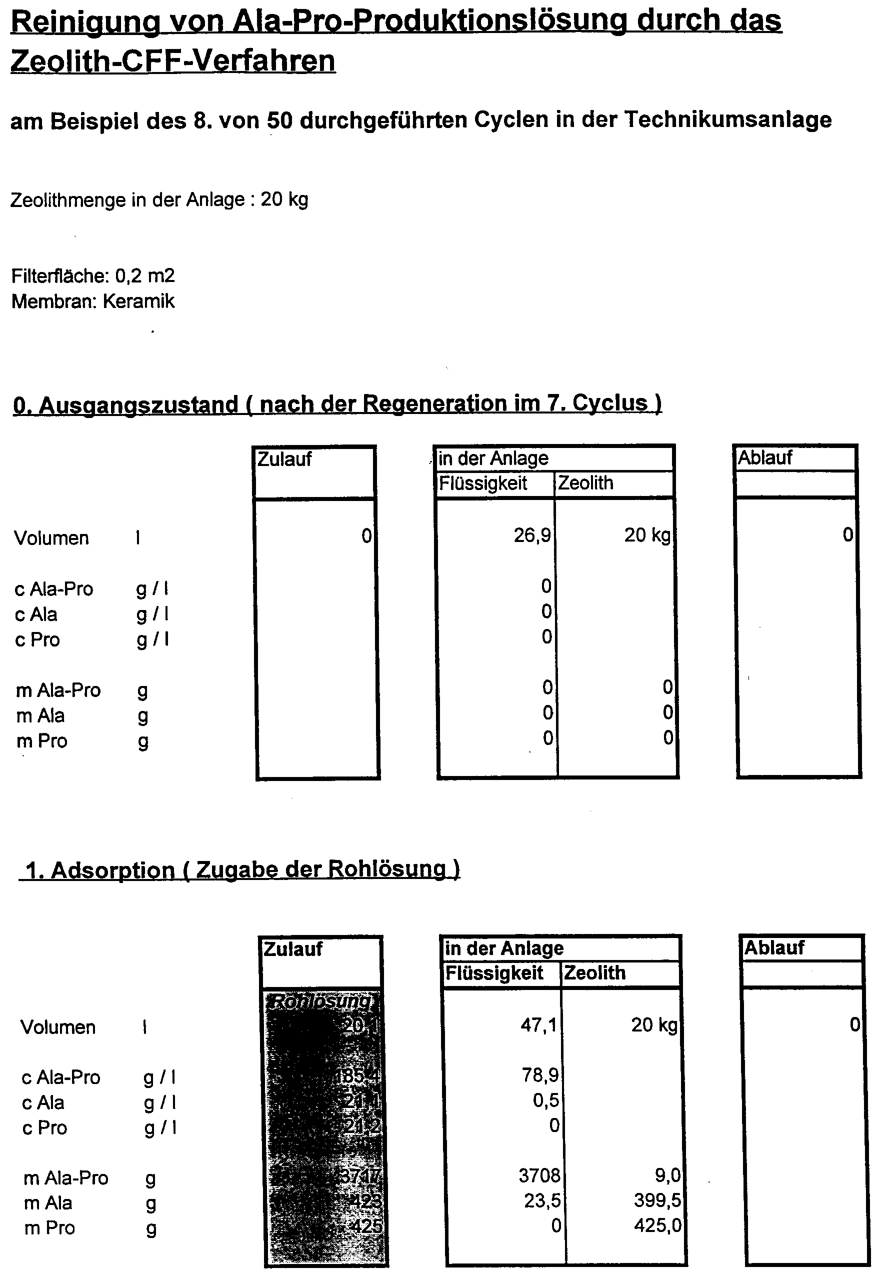

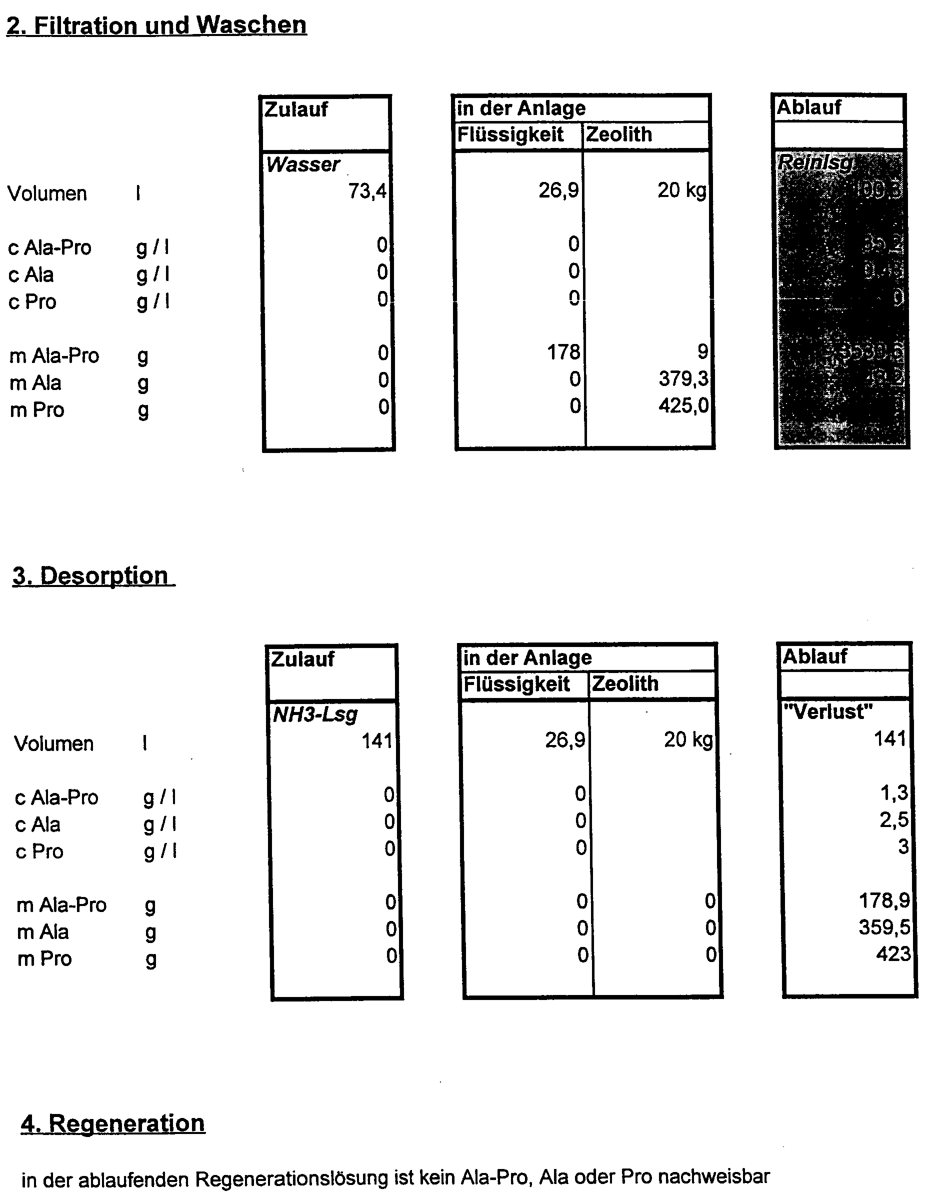

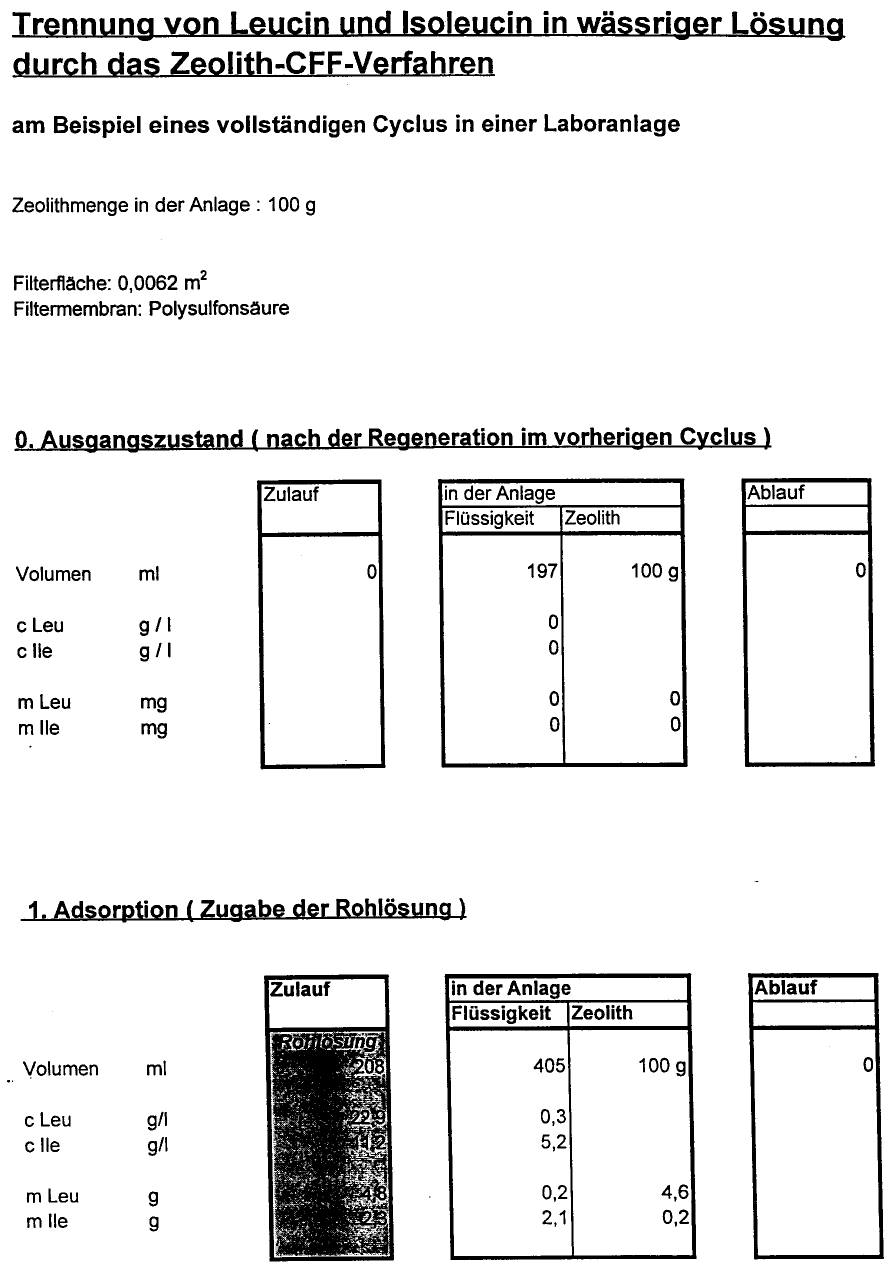

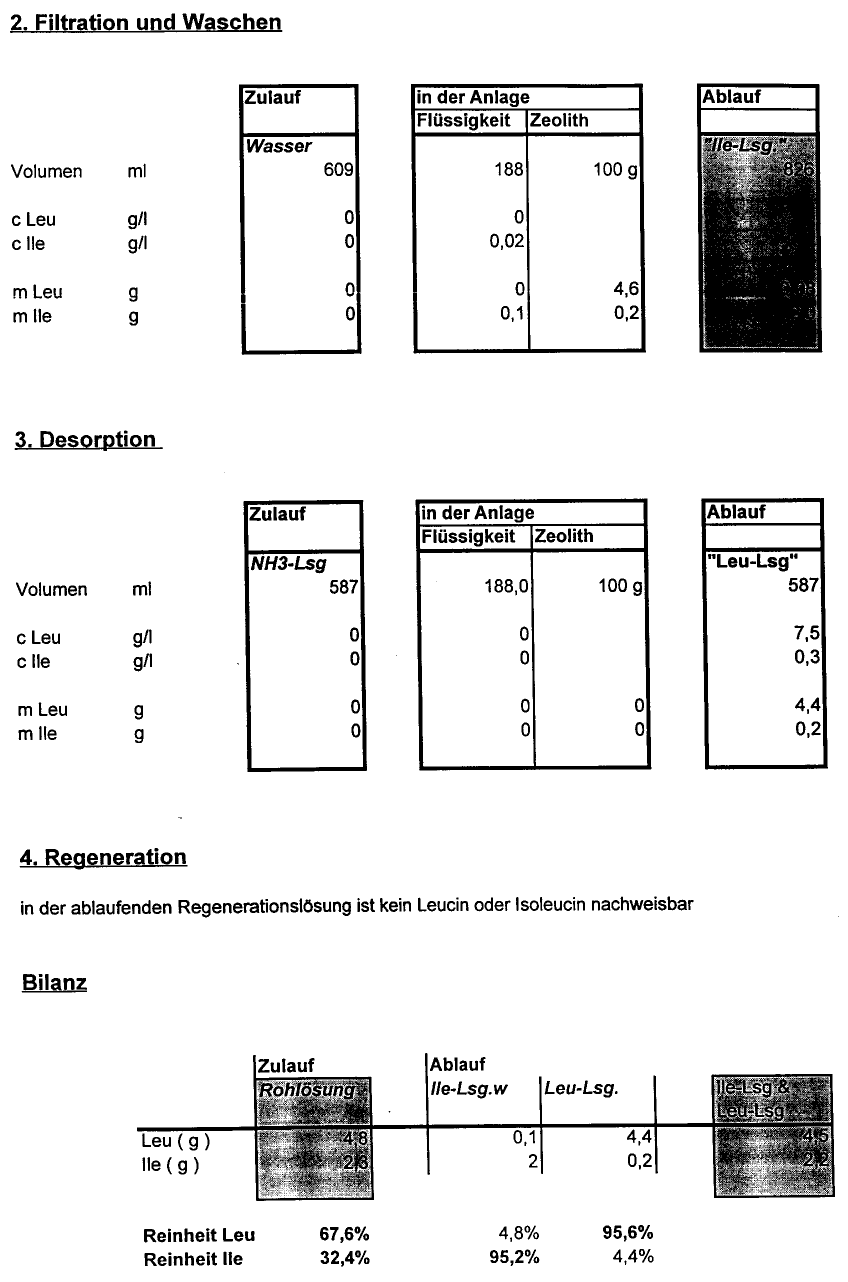

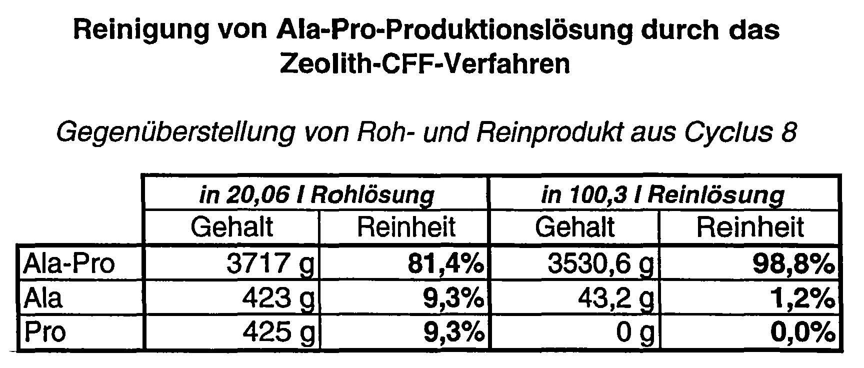

## Patentansprüche

1. Verfahren zur Trennung und/oder Abtrennung organischer, in Wasser löslicher Verbindungen, von denen der eine Teil an feinteiligen anorganischen Feststoffen adsorbiert wird, durch Adsorption dieses Anteils und Abtennung des beladenen Festoffs dadurch gekennzeichnet, daß man
a) die die organischen Verbindungen enthaltenden Lösungen mit einem geeigneten Feststoff in Kontakt bringt,dann eine Crossflow-Filtration durchführt, indem man
b) die entstehenden Suspensionen an einer porösen Fläche/Membran vorbeiströmen läßt, wobei man
c) eine Druckdifferenz zwischen der Überström- und der gegenüberliegenden Seite der Fläche/Membran einstellt,
d) dadurch ein Teil der die Fläche/Membran überströmenden von adsorbierbaren Verbindungen (a) ganz oder teilweise befreiten Lösung die Fläche/Membran quer zur Fließrichtung durchströmt (Filtratfluß)
e) man aus diesem Teil die nicht adsorbierten organischen Verbindungen (b) abtrennt,
f) den die Fläche/Membran nicht durchströmenden Teil der Lösung bzw. Suspension, gegebenenfalls nach Abtrennung eines Teils oder der Gesamtmenge des Feststoffs, wieder in den Kreislauf zur Adsorption der gegebenenfalls noch vorhandenen Restmengen an organischen Verbindungen zurückführt (Punkt a).

2. Verfahren gemäß Anspruch 1,
dadurch gekennzeichnet, daß man Feststoffe mit einem mittleren Partikeldurchmesser von 1 bis 20 µm einsetzt.

3. Verfahren gemäß den Ansprüchen 1 und 2,
dadurch gekennzeichnet, daß man 5 bis 60 Gew.-%ige Feststoffsuspensionen einsetzt.

4. Verfahren gemäß den Ansprüchen 1 bis 3,
dadurch gekennzeichnet, daß man als Feststoff einen Zeolith der Typen TA, FAU, MOR oder MSI einsetzt.

5. Verfahren gemäß den Ansprüchen 1 bis 3,
dadurch gekennzeichnet, daß man als Feststoff einen Titansilikalit einsetzt.

6. Verfahren gemäß den Ansprüchen 1 bis 3,
dadurch gekennzeichnet, daß man als Feststoffe anorganische Verbindungen mit regelmäßigen Mesoporen einsetzt.

7. Verfahren gemäß den Ansprüchen 1 bis 5,
dadurch gekennzeichnet, daß man einen Transmembrandruck von 0,2 bis 3 bar einstellt.

8. Verfahren gemäß den Ansprüchen 1 bis 7,
dadurch gekennzeichnet, daß man keramische oder organische Membranen/poröse Flächen mit Ultrafiltrations- oder Mikrofiltrationseigeschaften verwendet.

9. Verfahren gemäß den Ansprüchen 1 bis 8,
dadurch gekennzeichnet, daß man aus der die poröse Fläche/Membran durchströmenden Lösung die gewünschte Verbindung isoliert.

10. Verfahren gemäß den Ansprüchen 1 bis 9,
dadurch gekennzeichnet, daß man eine Reaktionslösung einsetzt, die nicht umgesetzte Ausgangsverbindungen adsorbieren läßt und das gewünschte Produkt aus der die poröse Fläche/Membran durchströmenden Lösung isoliert.

11. Verfahren gemäß den Ansprüchen 1 bis 9,
dadurch gekennzeichnet, daß man L-Leucin und L-Isoleucin voneinander trennt.

12. Verfahren gemäß Anspruch 10,
dadurch gekennzeichnet, daß man an den organischen adsorbierbare Aminosäuren und/oder Aminosulfonsäuren von diese enthaltende oder aus ihnen hergestellten Produkten abtrennt.

13. Verfahren gemäß Anspruch 12,dadurch gekennzeichnet, daß es sich bei den Produkten um Verbindungen handelt, die ebenso wie die als Edukte eingesetzten Aminosäuren und/oder Aminosulfonsäuren sowohl Amino- als auch Säuregruppen enthalten.

14. Verfahren gemäß den Ansprüchen 12 und 13
dadurch gekennzeichnet, daß es sich bei den Produkten um Verbindungen handelt, in denen mindestens zwei Aminosäuren und/oder Aminosulfonsäuren über eine Amidbindung verknüpft sind.

15. Verfahren gemäß den Ansprüchen 12 bis 14,
dadurch gekennzeichnet, daß die Amino- und Säuregruppen der Aminosäure und/oder Aminosulfonsäuren durch eine C₁-C₄-Alkylengruppen verbunden sind.

16. Verfahren gemäß den Ansprüchen 12 bis 15
dadurch gekennzeichnet, daß die als Produkte erhaltenen Di- oder Oligopeptide mindestens eine Aminosäure enthalten, die eine weitere funktionelle Gruppe besitzt.

17. Verfahren gemäß den Ansprüchen 12 bis 16,
dadurch gekennzeichnet, daß die als Produkte erhaltenen Di- oder Oligopeptide mindestens eine Aminosäure mit einer hydrophoben Seitenkette enthalten.

18. Verfahren gemäß den Ansprüchen 12 bis 17,
dadurch gekennzeichnet, daß die Di- oder Oligopeptide mindestens eine Aminosäure aufweisen, bei der die Aminogruppe als primäres, sekundäres, oder tertiäres Amin vorliegt.

19. Verfahren gemäß den Ansprüchen 12 bis 18,
dadurch gekennzeichnet, daß die Di- oder Oligopeptide Aminosäuren enthalten, deren Aminogruppe in der α-und/oder β-und/oder γ-Stellung vorliegt.

20. Verfahren gemäß den Ansprüchen 12 bis 19,
dadurch gekennzeichnet, daß man die Trennung in dem pH-Bereich durchführt, in dem die Adsorption an dem Feststoff stattfindet.

21. Verfahren gemäß Anspruch 1,
dadurch gekennzeichnet, daß man den regenerierten Feststoff und die aus diesem desorbierte Verbindung in einer wäßrigen Suspension mit dem entsprechenden pH-Wert an der porösen Fläche/Membran vorbeiströmen läßt und die desorbierte Verbindung durch Crossflow-Filtration von dem Feststoff abtrennt.
